(19) Europäisches Patentamt
European Patent Office
Office européen des brevets

(11) **EP 4 624 558 A1**

(12) **EUROPEAN PATENT APPLICATION**

(43) Date of publication:
**01.10.2025 Bulletin 2025/40**

(21) Application number: 24166335.0

(22) Date of filing: **26.03.2024**

(51) International Patent Classification (IPC):
*C11D 3/37* $^{(2006.01)}$

(52) Cooperative Patent Classification (CPC):
**C11D 3/3788; C11D 3/3707; C11D 17/0008;**
C11D 1/123; C11D 1/83; C11D 1/94; C11D 3/0026;
C11D 2111/14

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company**
**Cincinnati, OH 45202 (US)**

(72) Inventors:
• **EBERT, Sophia**
**64056 Ludwigshafen am Rhein (DE)**

• **HULSKOTTER, Frank**
**65824 Schwalbach am Taunus (DE)**
• **NIEBERLE, Joerg**
**67056 Ludwigshafen (DE)**
• **SAVEYN, Pieter Jan Maria**
**1853 Strombeek-Bever (BE)**
• **SI, Gang**
**Newcastle upon Tyne, NE12 9BZ (GB)**
• **VAN HECKE, Evelyne Johanna Lutgarde**
**1853 Strombeek-Bever (BE)**

(74) Representative: **P&G Patent Belgium UK**
**N.V. Procter & Gamble Services Company S.A.**
**Temselaan 100**
**1853 Strombeek-Bever (BE)**

(54) **LIQUID HAND DISHWASHING DETERGENT COMPOSITION**

(57)    The need for a dishwashing detergent composition which provides effective grease cleaning, and suds mileage in the presence of greasy soil, while also using cleaning ingredients having improved biodegradability, is met by formulating the detergent composition with an alkoxylated polyol comprising both propoxylation and ethoxylation, wherein the degree of propoxylation is higher than the degree of ethoxylation.

EP 4 624 558 A1

## Description

FIELD OF THE INVENTION

The invention relates to liquid hand dishwashing detergent compositions.

BACKGROUND OF THE INVENTION

Hand dishwashing detergents are widely used in households to clean dishes, utensils, and cookware. These detergents typically contain a combination of surfactants, solvents, builders, and polymers to facilitate grease removal and overall cleaning performance. However, many of the better performing ingredients, and especially cleaning polymers, are not biodegradable, or are relatively slowly biodegradable.

There is a growing demand for more environmentally friendly hand dishwashing detergent compositions with improved biodegradability. After use, the detergent compositions typically enter the household wastewater stream. While cleaning polymers improve the efficacy of grease removal, many can also take a long time to biodegrade in the waste-water stream.

Various attempts have been made to incorporate more biodegradable materials into detergent compositions. However, achieving a balance between enhanced biodegradability and effective cleaning, especially grease cleaning, remains a challenge.

Hence, a need remains for a dishwashing detergent composition which provides effective grease cleaning, and suds mileage in the presence of greasy soil, while also using cleaning ingredients having improved biodegradability.

JPH0820795A relates to a detergent composition which, when contained into a nozzled container, does not solidify or cause nozzle clogging and can be stably used over long, this detergent composition containing 0.1 to 10wt.% of 1mol glycerol or diglycerol adduct to 5 to 60mol of an alkylene oxide. US7938900B2 relates to an aqueous pigment preparation comprising: at least one organic and/or inorganic pigment, dispersants and/or surfactants, a trihydric or higher polyhydric alkoxylated alcohol, a polyglycol alkyl ether, if desired, hydrotropic oligomers and/or polymers, if desired, fats, oils or fatty acids, if desired, further additives typical in the preparation of aqueous pigment dispersions, and water. CN111518631A relates to a detergent composition which comprises ethoxylated and/or propoxylated mono-and/or polyols. JP7189610A relates to a solid detergent composition that has sufficient strength to clean the interior of cooking appliances equipped with an automatic cleaning system. WO2019/173688A relates to a solid, enzymatic detergent compositions and methods of making and using the same, the detergent compositions are particularly useful for cleaning medical and dental instruments, and for cleaning ware. EP3505609A relates to a detergent composition for the control and removal of biofilms, which have been formed on a surface, the detergent composition comprises a combination of a mannanase and a botanical active ingredient, so that the combined action of both components provides a high efficiency in the elimination of biofilms adhered on surfaces, both in open areas and in closed areas. JP6955746 relates to powder detergent compositions for automatic dishwasher for improving the fluidity of the powder detergent composition, suppressing the generation of scale, supplying the powder detergent quantitatively in small amounts, and having sufficient detergency even with a small amount of detergent. CN108690742A discloses a kind of optical mirror slip cleaning agents. CN106833953A relates to an antimicrobial deodorization eider down cleaning agent. WO2016/191238A relates to surfactant and detergent compositions containing propoxylated glycerine. ES2464872A relates to a composition for the control and elimination of biofilms, which have been developed on a surface, the composition comprises a detergent and a combination of three enzymes (lipase, protease and $\alpha$-amylase), so that the combined action of both components provides a high efficiency in the elimination of biofilms adhered on surfaces, both in open areas and in closed areas. JP5394133A relates to a liquid detergent composition suitable for textiles such as clothing. JP5324207A relates to a solid detergent for an automatic dishwasher. JP2008156250A relates to an alkylene oxide adduct of glycerol and its use as a crystallization inhibitor for aqueous solutions of alkylbenzene sulfonate. JP5213092A relates to a granular detergent composition, a method for producing the same, and a method for using the same, especially for washing textiles such as clothes, towels and sheets. WO2008/021971A relates to a method capable of supplying a detergent solution of a stable concentration to an automatic washing machine by using a detergent supply apparatus; a tablet detergent composition for an automatic washing machine that is used in the method; and a washing method using the composition. JP4554498A relates to a metal detergent composition, particularly a metal detergent composition mainly used in a metal band continuous production line of iron, aluminum, copper and the like. JP4810844A and JP4810843A relate to a detergent for body soap, hand soap, facial cleanser, hair shampoo and the like, having good foaming with no slimy feeling when rinsing.

SUMMARY OF THE INVENTION

The present invention relates to a liquid hand dishwashing detergent composition comprising: from 5% to 50% by weight of the composition of a surfactant system, wherein the surfactant system comprises anionic surfactant; and an alkoxylated polyol derived from a polyol core having essentially 3 to 5 -OH groups, wherein: at least one of the -OH groups is modified to form an alkylene oxide branch; each of the at least one alkylene oxide branches comprises ethylene oxide and propylene oxide; the weight average molecular weight (Mw) of the alkoxylated polyol is in the range of from 1.500 to 4.500 g/mol; and the propylene oxide content is at least 50% by weight in relation to the total weight of the alkoxylated polyol.

DETAILED DESCRIPTION OF THE INVENTION

Formulating liquid hand dishwashing detergent compositions to comprise an alkoxylated polyol, as described herein, provides a composition having improved biodegradability, while also proving improved greasy soil removal and suds mileage.

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (e.g., polyethylene, polypropylene, polystyrene, etc.) and wood.

The term "grease" or "greasy" as used herein means materials comprising at least in part (i.e., at least 0.5 wt% by weight of the grease in the material) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of the composition relating to suds character during the dishwashing process. The term "sudsing profile" of the composition includes initial suds volume generated upon dissolving and agitation, typically manual agitation, of the composition in the aqueous washing solution, and the retention of the suds during the dishwashing process. Preferably, hand dishwashing compositions characterized as having "good sudsing profile" tend to have high initial suds volume and/or sustained suds volume, particularly during a substantial portion of or for the entire manual dishwashing process. This is important as the consumer uses high suds as an indicator that enough composition has been dosed. Moreover, the consumer also uses the sustained suds volume as an indicator that enough active cleaning ingredients (e.g., surfactants) are present, even towards the end of the dishwashing process. The consumer usually renews the washing solution when the sudsing subsides. Thus, a low sudsing composition will tend to be replaced by the consumer more frequently than is necessary because of the low sudsing level.

"Easy rinsing" or "an easy rinsing profile" means that the foam generated during the main wash cycle can be rinsed faster and less water can be used to collapse the foam from the main wash cycle. Faster collapsing of the foam is preferred to reduce the amount of time spent rinsing and overall washing time, as well. Reducing the amount of water used to collapse the foam is preferred because it aids in water conservation.

It is understood that the test methods that are disclosed in the Test Methods Section of the present application must be used to determine the respective values of the parameters of Applicants' inventions as described and claimed herein.

All percentages are by weight of the total composition, as evident by the context, unless specifically stated otherwise. All ratios are weight ratios, unless specifically stated otherwise, and all measurements are made at 25°C, unless otherwise designated.

Liquid hand dishwashing detergent composition

The composition is a liquid composition, which is a liquid hand dishwashing composition, and hence is in liquid form. The liquid hand dishwashing composition is preferably an aqueous composition. As such, the composition can comprise from 50% to 85%, preferably from 50% to 75%, by weight of the total composition of water.

The composition may have a pH greater than or equal to 6.0, or a pH of from 6.0 to 12.0, preferably from 7.0 to 11.0, more preferably from 7.5 to 10.0, measured as a 10% aqueous solution in demineralized water at 20°C.

The composition of the present invention can be Newtonian or non-Newtonian, preferably Newtonian, over the usage shear rate range which is typically from 0.1 s$^{-1}$ to 100 s$^{-1}$. Preferably, when Newtonian, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof, over the typical usage shear rate range.

Alkoxylated polyol

The liquid hand dishwashing detergent composition comprises at least one alkoxylated polyol. For sake of clarity, the term "alkoxylated polyols" refers to compounds that are derived from polyols, herein called the "polyol core", but do not necessarily possess any non-modified -OH groups. At least one of the -OH groups of the polyol core is substituted with an alkylene oxide branch. Hence, the alkoxylated polyol comprises at least one side chain which is an alkylene oxide branch, added to an -OH group of the polyol core.

The side chains comprise, and preferably consist of ethylene oxides and propylene oxides. The side chains preferably end with an -OH group but may alternatively be capped, such as with a $C_1$ to $C_{20}$ alkyl group, preferably $C_1$ to $C_6$ alkyl group, more preferably $C_1$ alkyl group, such as a methyl group.

The term "alkylene oxide branch", as used herein, refers to a sub-structure of the alkoxylated polyols preferably comprising, essentially consisting of or consisting of a plurality of EO units and PO units.

The presence of alkylene oxides within or next to the (predominately) hydrophobic polyol core leads to an amphiphilic nature and thus to excellent cleaning properties for the alkoxylated polyols, of use in the present invention.

The alkoxylated polyol is derived from a polyol core having essentially 3 to 5 -OH groups. The polyol core can have essentially 4 to 5 -OH groups, preferably 4 -OH groups. The term "-OH group", as used herein, refers to hydroxyl groups, in particular alcohol groups. This also includes -OH groups in the context of aromatic structures, such as phenols. The term includes all alcohol groups independent of the status of its carbon atom. Thus, in the sense of the present invention primary, secondary as well as tertiary alcohols fall within the meaning of "-OH group". Preferably, the polyol core at least comprises two "terminal" primary alcohol groups. In addition to these two primary alcohols, the polyol may further possess two or three secondary alcohol groups. Not included within the scope of the term "-OH group" are -OH groups that are part of carboxylic acids.

Preferably, the alkoxylated polyols of the invention are based on polyols that have four to five -OH groups in total. The polyol core used to prepare such alkoxylated polyols of use in the invention may be a monomer or may be oligo- or polymer build up by an assembly process comprising -OH groups containing subunits. Also, in case the polyol core is based on an oligomer or polymer the total number of -OH groups is four to five, too. For sake of clarity, this means that the number of -OH groups in the inventive compound is not restricted to only four and five but can also be every decimal number between four and five.

For example, diglycerol possesses four -OH groups and triglycerol possesses five -OH groups. The skilled person understands that a polyglycerol (n = 2 to 3) mixture of diglycerol and triglycerol can be prepared, wherein the (population of) polyol has a total number of -OH groups that lies between four and five. Depending on the ratio of diglycerol and the ratio of triglycerol any decimal number between four and five can be adjusted. Thus, in preferred embodiments, the alkoxylated polyol of the invention has 4; 4.1; 4.2; 4.3; 4.4; 4.5; 4.6; 4.7; 4.8; 4.9 or 5 -OH groups. In more preferred embodiments, the number of -OH groups in the alkoxylated polyol refers to an average number of a mixture of alkoxylated polyols each having mainly four or five -OH groups.

Thus, the alkoxylated polyols of the invention may be a homomeric or heteromeric group of molecules based on polyols that have four to five -OH groups.

At least one of the -OH groups is modified to form an alkylene oxide branch. Each of the at least one alkylene oxide branches comprises ethylene oxide and propylene oxide.

Each of the alkylene oxide branches can comprise, preferably consist of, on average: at least 2 ethylene oxide units (EOs), preferably at least 3 EOs and more preferably at least 4 EOs; and at least 6 polypropylene oxide units (POs), preferably at least 7 POs and more preferably at least 8 POs.

Each of the alkylene oxide branches can comprise, preferably consist of, on average: not more than 12 EOs, preferably not more than 10 EOs and more preferably not more than 8 EOs; and not more than 25 POs, preferably not more than 17 POs and more preferably not more than 14 POs.

The skilled person will understand that the polyols may also be alkoxylated with other alkoxylations in addition to ethylene oxide and propoxylene oxide, though such alkoxylated polyols are less preferred. In this context, butylene oxide is mentioned. Further, the skilled person is also well-aware of helpful modifications of the alkoxy chain, such as modifications with lactones or hydroxy carbon acid as described in WO2021165468 A.

It is noted that all such numbers are numbers "on average" meaning that such numbers refer to the average number for such unit per -OH group calculated based on all -OH groups of an alkoxylated polyol.

The reactions leading to the inventive compounds are statistical reactions, meaning there is never just one chemically exactly defined compound present, but the alkoxylated polyol are typically always a mixture of slightly different structures, the difference of those structures clearly stemming from the facts that no reaction proceeds in exactly the same way and the same speed on all functional units, especially as the chemical reactivities of the functional units - here mainly those of the -OH groups, differs according to their environment, meaning that a primary alcohol group reacts differently than a secondary alcohol, and also the chemical environment of the groups may be different; this leads in an overall view to slightly deviating structures being present, and thus any compound of use in this invention being defined as in the various embodiments, and exemplified in the examples never is just one chemical compound, but always a mixture of slightly different compounds, having a statistical distribution. As the reactivities of those groups are not differing by a large extent, the differences are relatively small. Hence, defining an alkoxylated polyol by a prototypical member is a viable way of defining the structure. Also, defining the composition of the side chains by average numbers (including those variables defined based on the numbers of -OH groups being present in the alkoxylated polyols) is a useful way of defining the overall composition of any mixture herein defined as "an alkoxylated polyol" of use in the invention.

Therefore, unless otherwise indicated, the values, ranges and ratios given in the specification for the number of -OH groups and the molecular weight (Mn) relate to the number average values in heterogenic mixture of the synthesized alkoxylated polyols containing individual, slightly differing from each other chemical structures that result from the preparation methods, including the methods described herein. As known in polymer science, the polydispersity (weight-average molecular weight (Mw) / number-average molecular weight (Mn)) is then a measure for the (in) homogeneity within the mixture of different species in the alkoxylated polyol.

In line with the above, it is preferred that all -OH groups of the polyol core have been substituted with alkylene oxide branches and the alkylene oxide branches have slight variations in the amounts of the different alkoxylations. More preferably, the amount of EO units and/or PO units of different alkylene oxide branches within one molecule deviate from the average chain length by not more than 20%, not more than 15%, not more than 10% or not more than 5%, wherein the average chain length represents 100%.

Each of the alkylene oxide branches can comprise a block or random structure of ethylene oxide and propylene oxide, preferably a block structure, more preferably wherein each of the alkylene oxide branches are first propoxylated and then ethoxylated such that the propoxylation is closer to the polyol core. As such, the alkylene oxide branches can possess a block structure consisting of a PO block and an EO block, wherein the PO block has been reacted with the -OH groups of the polyol core. Alternatively, the alkylene oxide branches possess a block structure consisting of an EO block and a PO block, wherein the EO block is reacted with the -OH groups of the polyol core. It is more preferably that each of the alkylene oxide branches are first propoxylated and then ethoxylated such that the propoxylation is closer to the polyol core.

Preferably, all alkylene oxide branches attached to the -OH group of the polyol core have the same structure, in that sense that the number of EO and/or PO units per alkylene oxide branch is identical or, alternatively, the alkylene oxide branch structures vary slightly.

Without wishing being bound by the following explanation, a rationale exists to explain the resulting structures of the alkoxylated polyols: Due to the fact that the reactions in questions necessarily employed to prepare those structural orders of the side chains, and thus to prepare the specific inventive compounds, are reactions of quite reactive species which can lead under suitable conditions to almost complete and even "essentially complete" conversions of almost 100 % if not even 100%, the statistical deviation of the composition of the mixture of "alkoxylated polyols" in question is not that high, which in turn means that the structural order of the side chains do not show much deviation. Thus, it is a reliable assumption which can in principle be proven by sophisticated and thus time-consuming and expensive analytical means - such as multidimensional NMR-analyses - that it is generally accepted that such deviation exists; hence, no "specific alkoxylated polyol" will be "just one chemical compound of a clearly defined chemical structure", but clearly will consist of a a) mixture of slightly differing compounds, such differences lying in b) slight deviations may already in the structure of compound making up "the (unmodified) polyol" being employed for the further modification steps, and c) the slight deviations in the structural orders of the side chains may be attached by way of d) multi-step reactions due to e) variations in the chemical reactivities of the -OH groups, and f) due to slight inhomogeneities occurring in a commercial scale process. All of those factors a) to f) - to just mention a few important ones - lead to a "specific alkoxylated polyol" which is not one specific chemical compound but in fact a mixture of slightly differing compounds having an overall very similar chemical structure; thus, such structure is best described by average numbers for the variables and percentages for the amounts of the dominating structural order.

The alkylene oxide used to prepare the alkoxylated polyols may be derived from a fossil or non-fossil carbon source or even a mixture of the before mentioned. Preferably, the amount of non-fossil carbon atoms in the alkylene oxide branch is at least 10%, at least 20%, at least 40%, at least 70%, at least 95% or it solely comprises non-fossil derived carbon atoms. The skilled person is well-aware of commercial alkylene oxide products made of non-fossil carbon sources (these products are often sold as being sustainable, renewable or bio-based). For example, Croda International, Snaith, UK, sells ethylene oxide and related products based on bio-ethanol as ECO Range. Additionally, methods to prepare bio-based propylene oxide are also known (see Abraham, D. S., "Production of propylene oxide from propylene glycol" Master's Thesis University of Missouri-Columbia (2007) (75 pages)).

The polyol core can be a monomer, oligomer or polymer, wherein each of the oligomer and the polymer comprise a plurality of subunits, preferably the oligomer is a homooligomer or the polymer is a heteropolymer.

Preferably, the polyol core reacted with alkylene oxides does not comprise further functional groups (such as amines, amides, esters, carbonyl, carbonic acids, phosphate, sulfonate groups etc. and derivatives thereof) besides the -OH groups.

Preferably, the polyol core has a linear or branched, saturated or non-saturated backbone of carbon atoms. Preferably, the polyol core is derived from a polyol having a structure of

$$C_nH_{2n+2-m}(OH)_m,$$

wherein, n is a number selected from 3 to 20, preferably from 3 to 8, more preferably from 3 to 6, most preferably 3, 4, 5 and 6, and m is from 3 to 5, preferably 4 to 5, more preferably 4.

The polyol core can comprise 3 -OH groups. Such triols include glycerol and trimethylolpropane (TMP). Suitable triols can also possess four, five or six carbon atoms (C4, C5 and C6 triols), preferably linear C4, C5 and C6 triols. Such polyol cores having essentially three - OH groups can be selected from the group consisting of 1,2,3-butanetriol, 1,2,4-butanetriol, 1,2,3-pentanetriol, 1,2,4-pentanetriol, 1,2,5-pentanetriol, 1,3,4-pentanetriol, 1,3,5-pentanetriol, 2,3,4-pentanetriol, 1,2,3-hexanetriol, 1,2,4-hexanetriol, 1,2,5-hexanetriol, 1,2,6-hexanetriol, 1,3,4-hexanetriol, 1,3,5-hexanetriol, 1,3,6-hexanetriol, 1,4,5-hexanetriol and 2,3,4-hexanetriol.

The polyol core can comprise 4 -OH groups. Such polyols comprising 4 -OH include: meso-Erythritol, D-threitol, L-threitol, 1,2,5,6-hexanetetrol, pentaerythritol, diglycerol, and mixtures thereof.

The polyol core can comprise 5 -OH groups. Such polyols comprising 5 -OH include: xylitol, ribitol, arabitol, pentitol, triglycerol and mixtures thereof.

Particularly preferred are polyols which are selected from the group consisting of: trimethylolpropane, glycerol, meso-Erythritol, D-threitol, L-threitol, 1,2,5,6-hexanetetrol, pentaerythritol, xylitol, ribitol, arabitol, pentitol, diglycerol, triglycerol, and mixtures thereof.

Blends of polyols can be used. A preferred blend includes a blend of diglycerol and triglycerol.

The polyol core can have a molecular weight ranging from 80 to 500 g/mol, preferably from 100 to 300 g/mol and more preferably from 120 to 250 g/mol.

The weight average molecular weight (Mw) of the alkoxylated polyol is in the range of from 1.500 to 4.500 g/mol, or in the range of from 2.000 to 4.300 g/mol, preferably in the range of from 2.500 to 4.000 g/mol, more preferably in the range of from 3.000 to 3500 g/mol.

Methods to measure the weight of a polyol are well-known in the art and include mass spectrometry, mass photometry and static light scattering.

The person skilled in the art knows how to determine/measure the respective weight average molecular weight ($M_W$). This can be done, for example, by size exclusion chromatography (such as GPC, e.g., in combination with light scattering), mass spectrometry, or mass photometry. Preferably, $M_W$ values are determined by the method as follows: OECD (1996), Test No. 118: Determination of the Number-Average Molecular Weight and the Molecular Weight Distribution of Polymers using Gel Permeation Chromatography, OECD Guidelines for the Testing of Chemicals, Section 1, 2074-5753, OECD Publishing, Paris.

For the alkoxylated polyols described herein, the molecular weight (MW) can also be calculated from the used molar ratio of starting materials, since the alkoxylation reaction is essentially complete. Typically, the molecular weight (MW) of the alkoxylated polyol can be calculated using equation below:

The alkoxylated polyol can be described using the following nomenclature: (polyol core)$_1$[(PO/OH)$_x$(EO/OH)$_y$]$_m$, x refers to the average number of PO groups on each hydroxyl position of the polyol core, and y refers to the average number of EO groups on each hydroxyl position of the polyol core; m is the number of OH groups on the polyol used for the polyol core.

Using (Glycerol)$_1$/[(PO/OH)$_{12}$/(EO/OH)$_5$]$_3$ as an illustrative example: the polyol core is glycerol, which contains 3 OH groups. Therefore, the total mole of propoxylation (PO) is 36 (12 PO on each of 3 alkoxylated branches) and total mole of ethoxylation (EO) is 15 (5 EO on each of the 3 alkoxylated branches). The molecular weight of this specific polymer is then calculated as:

$$
\begin{aligned}
MW \quad &= MW \text{ of Glycerol} + MW \text{ of } 36 \text{ PO} + \text{total MW of } 15 \text{ EO} \\
&= 92.09 + (36 \times 58.08) + (15 \times 44.05) \\
&= 2843.72 \text{ g/mol}
\end{aligned}
$$

The % by weight of PO, or % by weight of EO, or % by weight of the polyol core in the alkoxylated polyol can be calculated follow the same principle.

The propylene oxide content is at least 50%, or from 50% to 96%, preferably from 56% to 90%, more preferably from 59% to 85%, more preferably from 62% to 80%, and most preferably between 65% and 75% by weight of the alkoxylated polyol.

When the molecular weight of the alkoxylated polyol is determined by gel permeation chromatography (GPC), the samples are prepared as follows: approx. 15 mg sample is dissolved in 10 ml eluent (tetrahydrofuran (THF) with 0.035 mol/L diethanolamine) for 1 hour at a temperature of 50°C. All sample solutions are filtered using a Chromafil® Xtra PTFE (0,20 $\mu$m) filter prior to injection. Sealed sample vials are placed into the auto sampler of an Agilent 1200 HPLC system or similar, set up with an isocratic pump, vacuum degasser, auto sampler and a column oven, with a Differential Refractive Index (DRI) and variable UltraViolet (UVW) Detector for detection. Data acquisition and data processing of conventionally SEC data is done using WinGPC Unichrom, build 6999 (Polymer Standard Services (PSS), now part of Agilent). A combination of a SDV guard (7,5 x 50 mm) column and 3 SDV columns (1000A, 100000A and 1000000A, all 7,5 x 300 mm) supplied by PSS are put in series at 60°C. The eluent was fed at a flow rate of 1 mL/min. 100$\mu$L of each sample solution was injected. The calibration was obtained by narrow molar mass distributed polyalkoxylene oxide (EO and PO mixtures) reference standards (supplied by Agilent) having a molar mass range of M= 160 till M = 1.378.000 g/mol. Molar masses outside this range were extrapolated.

"Mw" is the weight average molecular weight and "Mn" is number average molecular weight. The respective values of Mw and/or Mn can be determined as described within the experimental section below.

The molar mass distribution Mw/Mn obtained by GPC is equal to the polydispersity index (PDI), the PDI being without unit [g/mol / g/mol]).

Other preferred method to determine the molecular weight is measurement of the hydroxyl number.

The hydroxyl numbers were determined according to the phthalic anhydride method and are given in mg KOH/g.

The method is based on esterifying hydroxyl groups in polyols and polyol systems with phthalic acid anhydride at elevated temperatures and then titrating the excess phthalic acid anhydride with aqueous sodium hydroxide solution. Determination is done as follows:

An appropriate amount of sample is dissolved in 10 ml of a phthalic anhydride solution in pyridine (180 g/l) and stirred for 70 minutes at 105 °C. Subsequently, the air condenser used is rinsed with 5 ml of pyridine and the sample is titrated potentiometrically with 0.5 mol/l aqueous sodium hydroxide. A blank is performed analogously, but without the sample. The hydroxyl number is calculated from the difference in the volumes consumed. Any acids or bases in the sample must be taken into account.

The hydroxyl number is expressed as mg potassium hydroxide per one gram of sample. The molecular weight Mn of the polyol sample can then be calculated from the measured hydroxyl number according to following equation: molecular weight Mn in g/mol = [No of hydroxyl groups in starter] x [56100] / [measured hydroxyl number in mgKOH/g].

It is clear for a person skilled in the art, that for the alkoxylated polyols of this invention, the measured molecular weight by GPC, hydroxyl number or mass spectroscopy and calculated molecular weight are very consistent.

The alkoxylated polyol of the invention typically demonstrate at least 30%, preferably at least 40%, preferably at least 50%, preferably at least 60% or more preferably at least 75% biodegradability according to standard OECD 301F within 56 days, preferably within 28 days.

For the purposes of this invention, aerobic biodegradation in wastewater according to OECD 301F is expressed as a percentage of the theoretical oxygen demand (ThOD, which is measured by the elemental analysis of the compound of interest), which is needed to completely biodegrade the compound sample. Thus, the amount of oxygen taken up by the microbial population during biodegradation of the test substance (corrected for uptake by blank inoculum, run in parallel) is expressed as a percentage of ThOD. The obtained values are preferably measured in triplicate using the OECD 301F manometric respirometry method. The consumption of oxygen is determined by measuring the change in pressure in the apparatus using an OxiTop® C (Xylem 35 Analytics Germany Sales GmbH & Co KG). Details for the tests performed are given in the experimental section below.

The alkoxylate polyols, which are ethoxylated and propoxylated, as described herein, provide a combined core-shell-product (i.e., the alkoxylated polyol), in which the "core" is the polyol core, and the "shell" is the alkylene oxide branches, and exhibit a significant biodegradation value. Moreover, such alkoxylated polyols demonstrate cleaning performance which is comparable to that provided by current, non-biodegradable cleaning polymers.

The alkoxylated polyols of use in the present invention can be prepared using a process wherein a polyol essentially having 3 to 5 -OH groups is reacted with (i) ethylene oxide molecules and (ii) propylene oxide molecules, in the desired molar ratios. Whether the alkylene oxide branches comprise a block or random structure of ethylene oxide and propylene oxide, and when block, the order in which the blocks occur, depends on whether the ethylene oxide molecules and propylene oxide molecules are added together or sequentially, and the order of addition of the ethylene oxide molecules and propylene oxide molecules.

The conversion rate of the reaction step may be monitored and in preferred embodiments the conversion rate is at least 95%, preferably at least 99%, and even more preferably at least 99,5 % or even more. All other structural orders of the side chains as defined above but also the undefined structures resulting from non-controllable parameters are performed in this defined manner, leading - on statistical average - to a defined structural order directly derived from the way such reaction is performed.

The conversion rate of the reaction can be determined according to methods known to the skilled person, such as NMR-spectroscopy, such as 13C-NMR-spectroscopy and/or 1H NMR-spectroscopy.

For the reaction conditions such as catalysts, temperatures, duration, purification etc. of the reactions to produce the units of the side chains of the inventive alkoxylated polyols, the respective information within the disclosures EP3298120 A, JP2022056680 A and US7468348 B is fully encompassed into this recent disclosure by way of reference.

Typically, the alkoxylation is carried out in the presence of at least one catalyst. Within this single step reaction of the alkoxylation step, the catalyst is preferably a basic catalyst. Examples of suitable catalysts are alkali metal and alkaline earth metal hydroxides such as sodium hydroxide, potassium hydroxide and calcium hydroxide, alkali metal alkoxides, in particular sodium and potassium $C_1$-$C_4$-alkoxides, such as sodium methoxide, sodium ethoxide and potassium tert-butoxide, alkali metal and alkaline earth metal hydrides such as sodium hydride and calcium hydride, and alkali metal carbonates such as sodium carbonate and potassium carbonate. Preference is given to the alkali metal hydroxides and the alkali metal alkoxides, a particular preference being given to potassium hydroxide and sodium hydroxide. Typical use amounts for the base are from 0.05 to 10% by weight of final product, in particular from 0.05 to 2% by weight, based on the total amount of polyol and alkylene oxide.

Preferably, the alkoxylated polyol is further submitted to the following process steps of

a. purification using standard means such as steam distillation, thermal distillation, vacuum evaporation, including removal of all solvent, dialysis and/or
b. drying using standard drying means such as spray-, drum, paddle-, vacuum-drying means including agglomeration methods such as fluidized-bed-drying,

to obtain a purified solution, a purified liquid, a solid compound or a purified solid compound, respectively.

In case that after the reaction leading to the alkoxylated polyol residual educts (polyol and/or alkylene oxide) are present to a non-desirable extent, the resulting product mixture containng the alkoxylated polyol may be further purified by standard means to reduce the content of residual educts, but also to reduce the amount of possible by-products, reduce the amount(s) of the solvent(s) employed (i.e., to concentrate) or replace solvent(s) with other solvents. Such processes are known to a person of skill in this field.

Preferably, undesirable amounts of residual non-reacted educts are removed, preferably by means of distillative processes, more preferably by thermal distillative processes, which may additionally comprise the application of reduced pressure to increase the speed and/or the effectiveness of the removal.

In a preferred embodiment only the additional process step a) is employed.

The terms "essentially having" or "having essentially", as used interchangeably herein, with respect to the polyol core mean that the polyol core may comprise impurities or other types of polyols in an amount up to not more than 10% w/w, not more than 7% w/w, not more than 5% w/w, not more than 3% w/w, not more than 2% w/w, not more than 1% w/w, not more than 0.5% w/w or not more than 0.1% w/w.

Surfactant System

The liquid composition comprises from 5.0% to 50%, preferably from 6.0% to 40%, most preferably from 15% to 35%, by weight of the total composition of a surfactant system.

Anionic surfactant:

The surfactant system comprises an anionic surfactant. The surfactant system can comprise at least 40%, preferably from 50% to 90%, more preferably from 65% to 85% by weight of the surfactant system of the anionic surfactant. The surfactant system is preferably free of fatty acid or salt thereof, since such fatty acids impede the generation of suds.

Suitable anionic surfactants can be selected from the group consisting of: alkyl sulfate surfactant, alkyl alkoxy sulfate surfactant, alkyl sulfonate surfactant, alkyl sulfosuccinate and dialkyl sulfosuccinate ester surfactants, and mixtures thereof.

The anionic surfactant can comprise at least 70%, preferably at least 85%, more preferably 100% by weight of the anionic surfactant of alkyl sulfate anionic surfactant, alkyl alkoxy sulfate anionic surfactant, or a mixture thereof.

The mol average alkyl chain length of the alkyl sulfate anionic surfactant or the alkyl alkoxy sulfate anionic surfactant can be from 8 to 18, preferably from 10 to 14, more preferably from 12 to 14, most preferably from 12 to 13 carbon atoms, in order to provide a combination of improved grease removal and enhanced speed of cleaning.

The alkyl chain of the alkyl sulfate anionic surfactant or the alkyl alkoxy sulfate anionic surfactant can have a mol fraction of C12 and C13 chains of at least 50%, preferably at least 65%, more preferably at least 80%, most preferably at least 90%. Suds mileage is particularly improved, especially in the presence of greasy soils, when the C13/C12 mol ratio of the alkyl chain is at least 57/43, preferably from 60/40 to 90/10, more preferably from 60/40 to 80/20, most preferably from 60/40 to 70/30, while not compromising suds mileage in the presence of particulate soils.

The relative molar amounts of C13 and C12 alkyl chains in the alkyl sulfate anionic surfactant or the alkyl alkoxy sulfate anionic surfactant can be derived from the carbon chain length distribution of the surfactants. The carbon chain length distributions of the alkyl chains of the alkyl sulfate and alkyl alkoxy sulfate surfactants can be obtained from the technical data sheets from the suppliers for the surfactant or constituent alkyl alcohol. Alternatively, the chain length distribution and average molecular weight of the fatty alcohols, used to make the alkyl sulfate anionic surfactant or the alkyl alkoxy sulfate anionic surfactant, can also be determined by methods known in the art. Such methods include capillary gas chromatography with flame ionization detection on medium polar capillary column, using hexane as the solvent. The chain length distribution is based on the starting alcohol and alkoxylated alcohol. As such, the alkyl sulfate anionic surfactant should be hydrolyzed back to the corresponding alkyl alcohol and alkyl alkoxylated alcohol before analysis, for instance using hydrochloric acid.

The alkyl alkoxy sulfate surfactant can have an average degree of alkoxylation of less than 3.5, preferably less than 2.0, more preferably 1.0 or less. Alternatively, the alkyl alkoxy sulfate surfactant can have an average degree of alkoxylation of less than 3.5, preferably from 0.3 to 2.0, more preferably from 0.5 to 0.9, in order to improve low temperature physical stability and improve suds mileage of the compositions of the present invention. When alkoxylated, ethoxylation is preferred.

The average degree of alkoxylation is the mol average degree of alkoxylation (i.e., mol average alkoxylation degree) of all the alkyl sulfate anionic surfactant. Hence, when calculating the mol average alkoxylation degree, the mols of non-alkoxylated sulfate anionic surfactant are included:

Mol average alkoxylation degree = (x1 * alkoxylation degree of surfactant 1 + x2 * alkoxylation degree of surfactant 2 + ....) / x1 + x2 + ....)

where x1, x2, ... are the number of moles of each alkyl (or alkoxy) sulfate anionic surfactant of the mixture and alkoxylation degree is the number of alkoxy groups in each alkyl sulfate anionic surfactant.

Preferred alkyl alkoxy sulfates are alkyl ethoxy sulfates.

The alkyl sulfate anionic surfactant and the alkyl alkoxy sulfate anionic surfactant can have a weight average degree of branching of at least 10%, preferably from 20% to 60%, more preferably from 25% to 45%. Alternatively, the alkyl sulfate anionic surfactant and the alkyl alkoxy sulfate anionic surfactant can have a weight average degree of branching of less than 10%, preferably the alkyl sulfate anionic surfactant and the alkyl alkoxy sulfate anionic surfactant are free of branching.

The alkyl sulfate anionic surfactant and the alkyl alkoxy sulfate anionic surfactant can comprise at least 5%, preferably at least 10%, most preferably at least 25%, by weight of the surfactant, of branching on the C2 position (as measured counting carbon atoms from the sulfate group for non-alkoxylated alkyl sulfate anionic surfactants and counting from the alkoxy-group furthest from the sulfate group for alkoxylated alkyl sulfate anionic surfactants). More preferably, greater than 75%, even more preferably greater than 90%, by weight of the total branched alkyl content consists of C1-C5 alkyl moiety, preferably C1-C2 alkyl moiety. It has been found that formulating the inventive compositions using alkyl sulfate surfactants or alkyl alkoxy sulfate surfactants having the aforementioned degree of branching results in improved low temperature stability. Such compositions require less solvent in order to achieve good physical stability at low temperatures. As such, the compositions can comprise lower levels of organic solvent, such as less than 5.0% by weight of the liquid composition of organic solvent, while still having improved low temperature stability. Higher surfactant branching also provides faster initial suds generation, but typically less suds mileage. The weight average branching, described herein, has been found to provide improved low temperature stability, initial foam generation and suds longevity.

The weight average degree of branching for an anionic surfactant mixture can be calculated using the following formula:

Weight average degree of branching (%) = [(x1 * branched alcohol 1 in alcohol 1 + x2 * wt% branched alcohol 2 in alcohol 2 + ....) / (x1 + x2 + ....)] * 100 where x1, x2, ... are the weight in grams of each alcohol in the total alcohol mixture of the alcohols which were used as starting material before (alkoxylation and) sulfation to produce the alkyl (alkoxy) sulfate anionic surfactant. In the weight average degree of branching calculation, the weight of the alkyl alcohol used to form the alkyl sulfate anionic surfactant which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionization detection on medium polar capillary column, using hexane as the solvent. The weight average degree of branching and the distribution of branching is based on the starting alcohol used to produce the alkyl sulfate anionic surfactant.

Suitable counterions include alkali metal cation earth alkali metal cation, alkanolammonium or ammonium or substituted ammonium, but preferably sodium.

Suitable examples of commercially available alkyl sulfate anionic surfactants include, those derived from alcohols sold under the Neodol® brand-name by Shell, or the Lial®, Isalchem®, and Safol® brand-names by Sasol, or some of the natural alcohols produced by The Procter & Gamble Chemicals company. The alcohols can be blended in order to achieve the desired mol fraction of C12 and C13 chains and the desired C13/C12 ratio, based on the relative fractions of C13 and C12 within the starting alcohols, as obtained from the technical data sheets from the suppliers or from analysis using methods known in the art.

The performance can be affected by the width of the alkoxylation distribution of the alkoxylated alkyl sulfate anionic surfactant, including grease cleaning, sudsing, low temperature stability and viscosity of the finished product. The alkoxylation distribution, including its broadness can be varied through the selection of catalyst and process conditions when making the alkoxylated alkyl sulfate anionic surfactant.

If ethoxylated alkyl sulfate is present, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-$\alpha$-hydroxy-7-oxo stereoisomer-mixtures of cholinic acid, 3-(N- methyl amino)-L-alanine, and mixtures thereof.

Anionic alkyl sulfonate or sulfonic acid surfactants suitable for use herein include the acid and salt forms of alkylbenzene sulfonates, alkyl ester sulfonates, primary and secondary alkane sulfonates such as paraffin sulfonates, alfa or internal olefin sulfonates, alkyl sulfonated (poly)carboxylic acids, and mixtures thereof. Suitable anionic sulfonate or sulfonic acid surfactants include: C5-C20 alkylbenzene sulfonates, more preferably C10-C16 alkylbenzene sulfonates, more preferably C11-C13 alkylbenzene sulfonates, C5-C20 alkyl ester sulfonates especially C5-C20 methyl ester sulfonates, C6-C22 primary or secondary alkane sulfonates, C5-C20 sulfonated (poly)carboxylic acids, and any mixtures thereof, but preferably C11-C13 alkylbenzene sulfonates. The aforementioned surfactants can vary widely in their 2-phenyl isomer content. Compared with sulfonation of alpha olefins, the sulfonation of internal olefins can occur at any position since the double bond is randomly positioned, which leads to the position of hydrophilic sulfonate and hydroxyl groups of IOS in the middle of the alkyl chain, resulting in a variety of twin-tailed branching structures. Alkane sulfonates include paraffin sulfonates and other secondary alkane sulfonate (such as Hostapur SAS60 from Clariant).

Alkyl sulfosuccinate and dialkyl sulfosuccinate esters are organic compounds with the formula MO3SCH(CO2R')CH2CO2R where R and R' can be H or alkyl groups, and M is a counter-ion such as sodium (Na). Alkyl sulfosuccinate and dialkyl sulfosuccinate ester surfactants can be alkoxylated or non-alkoxylated, preferably non-alkoxylated. The surfactant system may comprise further anionic surfactant. However, the composition preferably comprises less than 30%, preferably less than 15%, more preferably less than 10% by weight of the surfactant system of further anionic surfactant. Most preferably, the surfactant system comprises no further anionic surfactant, preferably no other anionic surfactant than alkyl sulfate anionic surfactant.

Co-Surfactant:

In order to improve foaming, as well as surfactant packing after dilution and hence improve suds mileage, the surfactant system can comprise a co-surfactant. The co-surfactant comprises an amphoteric surfactant which is an amine oxide surfactant, wherein the amine oxide surfactant has the formula: R1N(R2)(R3)O, wherein R1 is a C8-C18 alkyl, and the R2 and R3 moieties are selected from the group consisting of C1-C3 alkyl groups, C1-C3 hydroxyalkyl groups, and mixtures thereof.

The anionic surfactant to the amine oxide amphoteric co-surfactant weight ratio can be from 1:1 to 8:1, preferably from 2:1 to 5:1, more preferably from 2.5:1 to 4:1.

The composition preferably comprises from 0.1% to 20%, more preferably from 0.5% to 15% and especially from 2% to 10% by weight of the composition of the amine oxide amphoteric co-surfactant.

The surfactant system of the composition of the present invention preferably comprises up to 50%, preferably from 10% to 40%, more preferably from 15% to 35%, by weight of the surfactant system of the amine oxide amphoteric co-surfactant.

The amine oxide amphoteric surfactant can be linear or branched, though linear are preferred. Suitable linear amine oxides are typically water-soluble.

R2 and R3 can be selected from the group consisting of methyl, ethyl, propyl, isopropyl, 2-hydroxethyl, 2-hydroxypropyl and 3-hydroxypropyl, and mixtures thereof, though methyl is preferred for one or both of R2 and R3, preferably both. The linear amine oxide surfactants, in particular, may include linear C10-C18 alkyl dimethyl amine oxides and linear C8-C12 alkoxy ethyl dihydroxy ethyl amine oxides.

Preferably, the amine oxide surfactant is selected from the group consisting of alkyl dimethyl amine oxide and mixtures thereof. Alkyl dimethyl amine oxides are particularly preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, or mixtures thereof. C12-C14 alkyl dimethyl amine oxide is particularly preferred, especially linear C12-14 alkyl dimethyl amine oxide. Suitable linear C12-C14 alkyl dimethyl amine oxide surfactants can be derived from natural alcohols, especially coconut oil derived alcohols, or can be derived from synthetic alcohols through the Ziegler process.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein, "symmetric" means that $|n1 - n2|$ is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-C3 alkyl, a C1-C3 hydroxyalkyl group, or a polyethylene oxide group containing an average of from about 1 to about 3 ethylene oxide groups. Preferably, the two moieties are selected from a C1-C3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

The composition can comprise further co-surfactant selected from zwitterionic surfactants including betaine surfactants. However, preferably, the composition comprises less than 5.0%, preferably less than 1.0%, more preferably less than 0.5% by weight of the composition of such betaine surfactants, and is most preferably free of betaine surfactant.

If present, suitable betaine surfactants include alkyl betaines, alkylamidobetaine, amidazoliniumbetaine, sulfobetaine (INCI Sultaines) as well as the phosphobetaine, and preferably meets formula (I):

$$R1\text{-}[CO\text{-}X(CH2)_n]_x\text{-}N^+(R2)(R3)\text{-}(CH_2)_m\text{-}[CH(OH)\text{-}CH_2]_y\text{-}Y\text{-}$$

Wherein in formula (I),

R1 is selected from the group consisting of: a saturated or unsaturated C6-22 alkyl residue, preferably C8-18 alkyl residue, more preferably a saturated C10-16 alkyl residue, most preferably a saturated C12-C14 alkyl residue;

X is selected from the group consisting of: NH, NR4 wherein R4 is a C1-4 alkyl residue, O, and S,

n is an integer from 1 to 10, preferably 2 to 5, more preferably 3,

x is 0 or 1, preferably 1,

R2 and R3 are independently selected from the group consisting of: a C1-4 alkyl residue, hydroxy substituted such as a hydroxyethyl, and mixtures thereof, preferably both R2 and R3 are methyl,

m is an integer from 1 to 4, preferably 1, 2 or 3,

y is 0 or 1, and

Y is selected from the group consisting of: COO, $SO_3$, OPO(ORS)O or P(O)(OR5)O, wherein R5 is H or a C1-4 alkyl residue.

Preferred betaines are the alkyl betaines of formula (Ia), the alkyl amido propyl betaine of formula (Ib), the sulfobetaine of formula (Ic) and the amido sulfobetaine of formula (Id):

$$R1-N^+(CH_3)_2-CH_2COO- \qquad (Ia)$$

$$R1-CO-NH-(CH_2)_3-N^+(CH_3)_2-CH_2COO- \qquad (Ib)$$

$$R1-N^+(CH_3)_2-CH_2CH(OH)CH_2SO_3^- \qquad (Ic)$$

$$R1-CO-NH-(CH_2)_3-N^+(CH_3)_2-CH_2CH(OH)CH_2SO_3^- \qquad (Id)$$

in which R1 has the same meaning as in formula (I). Particularly preferred are the carbobetaines [i.e., where Y=COO in formula (I)] of formulae (Ia) and (Ib), more preferred are the alkylamidobetaine of formula (Ib).

Suitable betaines can be selected from the group consisting or [designated in accordance with INCI]: capryl/-capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine is particularly preferred.

Nonionic Surfactant:

The surfactant system can further comprise a nonionic surfactant. Suitable nonionic surfactants include alkoxylated alcohol nonionic surfactants, alkyl polyglucoside nonionic surfactants, and mixtures thereof, preferably alkoxylated alcohol nonionic surfactants.

Alkoxylated alcohol nonionic surfactant:

If present, the surfactant system of the composition of the present invention can comprise from 0.1% to 10%, preferably from 2.0% to 9.0%, more preferably from 4.0% to 8.0% by weight of the detergent composition, of an alkoxylated alcohol non-ionic surfactant.

Preferably, the alkoxylated alcohol non-ionic surfactant is a linear or branched, primary or secondary alkyl alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol.

Alkyl polyglucoside nonionic surfactant:

If present, the alkyl polyglucoside can be present in the surfactant system at a level of from 0.1% to 10%, preferably from 2.0% to 9.0%, more preferably from 4.0% to 8.0% by weight of the detergent composition. Alkyl polyglucoside nonionic surfactants are typically more sudsing than other nonionic surfactants such as alkyl ethoxlated alcohols.

A combination of alkylpolyglucoside and anionic surfactant especially alkyl sulfate anionic surfactant, has been found to improve polymerized grease removal, suds mileage performance, reduced viscosity variation with changes in the surfactant and/or system, and a more sustained Newtonian rheology.

The alkyl polyglucoside surfactant can be selected from C6-C18 alkyl polyglucoside surfactant. The alkyl polyglucoside surfactant can have a number average degree of polymerization of from 0.1 to 3.0, preferably from 1.0 to 2.0, more preferably from 1.2 to 1.6. The alkyl polyglucoside surfactant can comprise a blend of short chain alkyl polyglucoside surfactant having an alkyl chain comprising 10 carbon atoms or less, and mid to long chain alkyl polyglucoside surfactant having an alkyl chain comprising greater than 10 carbon atoms to 18 carbon atoms, preferably from 12 to 14 carbon atoms.

Short chain alkyl polyglucoside surfactants have a monomodal chain length distribution between C8-C10, mid to long chain alkyl polyglucoside surfactants have a monomodal chain length distribution between C10-C18, while mid chain alkyl polyglucoside surfactants have a monomodal chain length distribution between C12-C14. In contrast, C8 to C18 alkyl polyglucoside surfactants typically have a monomodal distribution of alkyl chains between C8 and C18, as with C8 to C16 and the like. As such, a combination of short chain alkyl polyglucoside surfactants with mid to long chain or mid chain alkyl polyglucoside surfactants have a broader distribution of chain lengths, or even a bimodal distribution, than non-blended C8 to C18 alkyl polyglucoside surfactants. Preferably, the weight ratio of short chain alkyl polyglucoside surfactant to long chain alkyl polyglucoside surfactant is from 1:1 to 10:1, preferably from 1.5:1 to 5:1, more preferably from 2:1 to 4:1. It has been found that a blend of such short chain alkyl polyglucoside surfactant and long chain alkyl polyglucoside surfactant results in faster dissolution of the detergent solution in water and improved initial sudsing, in combination with improved suds stability.

C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Glucopon® 215UP is a preferred short chain APG surfactant. Glucopon® 600CSUP is a preferred mid to long chain APG surfactant.

In preferred compositions, the surfactant system can comprise an alkyl sulfate anionic surfactant having an average degree of branching of less than 10% and alkyl polyglucoside nonionic surfactant.

Divalent salts

The composition preferably comprises a divalent metal salt, preferably a salt of Calcium or Magnesium ($Ca^{2+}$ or $Mg^{2+}$ salt). Suitable divalent salts include magnesium and/or calcium salts of: chlorides, sulfates, carbonates, bicarbonates, linear alkyl benzene sulfonic acid, and mixtures thereof, with magnesium salts being particularly preferred. Magnesium salts of chlorides, sulfates, linear alkyl benzene sulfonic acid, and mixtures thereof are particularly preferred, more particularly magnesium salts of chlorides, sulfates, and mixtures thereof, with magnesium chloride being most preferred.

If calcium salts are present, the magnesium ions and calcium ions are preferably present in a molar ratio of 1:1 or greater, preferably 1.5: 1 or greater, preferably 2: 1 or greater.

Compositions of the present invention, which further comprise such divalent salts have been found to improve cleaning as well as reduce the slipperiness of the dishware after they have been cleaned with such compositions. It is believed that some residual anionic surfactant remains on the dishware, and the presence of the divalent ions reduces the electrostatic interaction between the residual anionic surfactant, both improving cleaning and reducing slipperiness, especially when the dishware is washed using soft water having a hardness of less than 1.25 mmol/l calcium equivalence.

The divalent salts are preferably water-soluble. As used herein, the term "water-soluble" refers to a compound that can be dissolved in water at a concentration of more than 1.0% by weight in distilled water at 21°C.

Further ingredients

The composition can comprise further ingredients such as those selected from: triblock copolymers, hydrotropes, organic solvents, other adjunct ingredients such as those described herein, and mixtures thereof.

Triblock copolymer:

The composition of the invention can comprise a triblock copolymer. The triblock co-polymers can be present at a level of from 1% to 20%, preferably from 3% to 15%, more preferably from 5% to 12%, by weight of the total composition. Suitable triblock copolymers include alkylene oxide triblock co-polymers, defined as a triblock co-polymer having alkylene oxide moieties according to Formula (I): $(EO)_x(PO)_y(EO)_x$, wherein EO represents ethylene oxide, and each x represents the number of EO units within the EO block. Each x can independently be on average of from 5 to 50, preferably from 10 to 40, more preferably from 10 to 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y can on average be from between 28 to 60, preferably from 30 to 55, more preferably from 30 to 48.

Preferably the triblock co-polymer has a ratio of y to each x of from 3:1 to 2:1. The triblock co-polymer preferably has a ratio of y to the average x of 2 EO blocks of from 3:1 to 2:1. Preferably the triblock co-polymer has an average weight percentage of total E-O of between 30% and 50% by weight of the tri-block co-polymer. Preferably the triblock co-polymer has an average weight percentage of total PO of between 50% and 70% by weight of the triblock co-polymer. It is understood that the average total weight % of EO and PO for the triblock co-polymer adds up to 100%. The triblock co-polymer can have an average molecular weight of between 2060 and 7880, preferably between 2620 and 6710, more preferably between 2620 and 5430, most preferably between 2800 and 4700. Average molecular weight is determined using a 1H NMR spectroscopy (see Thermo scientific application note No. AN52907).

Triblock co-polymers have the basic structure ABA, wherein A and B are different homopolymeric and/or monomeric units. In this case A is ethylene oxide (EO) and B is propylene oxide (PO). Those skilled in the art will recognize the phrase "block copolymers" is synonymous with this definition of "block polymers".

Triblock co-polymers according to Formula (I) with the specific EO/PO/EO arrangement and respective homopolymeric lengths have been found to enhances suds mileage performance of the liquid hand dishwashing detergent composition in the presence of greasy soils and/or suds consistency throughout dilution in the wash process.

Suitable EO-PO-EO triblock co-polymers are commercially available from BASF such as Pluronic® PE series, and from the Dow Chemical Company such as Tergitol™ L series. Particularly preferred triblock co-polymer from BASF are sold under the tradenames Pluronic® PE6400 (MW ca 2900, ca 40wt% EO) and Pluronic® PE 9400 (MW ca 4600, 40 wt% EO). Particularly preferred triblock co-polymer from the Dow Chemical Company is sold under the tradename Tergitol™ L64 (MW ca 2700, ca 40 wt% EO).

Preferred triblock co-polymers are readily biodegradable under aerobic conditions.

Cyclic polyamine:

The composition can comprise a cyclic polyamine having amine functionalities that helps cleaning. The composition of the invention preferably comprises from 0.1% to 3%, more preferably from 0.2% to 2%, and especially from 0.5% to 1%, by weight of the composition, of the cyclic polyamine.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the cleaning composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile throughout the dishwashing process when formulated together with the surfactant system of the composition of the present invention.

Suitable cyclic polyamines can be supplied by BASF, under the Baxxodur tradename, with Baxxodur ECX-210 being particularly preferred.

A combination of the cyclic polyamine and magnesium sulfate is particularly preferred. As such, the composition can further comprise magnesium sulfate at a level of from 0.001 % to 2.0 %, preferably from 0.005 % to 1.0 %, more preferably from 0.01 % to 0.5 % by weight of the composition.

Salt, hydrotrope, organic solvent:

The composition of the present invention may further comprise at least one active selected from the group consisting of: i) a salt, ii) a hydrotrope, iii) an organic solvent, and mixtures thereof.

The composition of the present invention may comprise from about 0.05% to about 2%, preferably from about 0.1% to about 1.5%, or more preferably from about 0.5% to about 1%, by weight of the total composition of a salt, preferably a monovalent or divalent inorganic salt, or a mixture thereof, more preferably selected from: sodium chloride, sodium sulfate, and mixtures thereof. Sodium chloride is most preferred.

The composition of the present invention may comprise from about 0.1% to about 10%, or preferably from about 0.5% to about 10%, or more preferably from about 1% to about 10% by weight of the total composition of a hydrotrope or a mixture thereof, preferably sodium cumene sulfonate.

The composition can comprise from about 0.1% to about 10%, or preferably from about 0.5% to about 10%, or more preferably from about 1% to about 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol, is the preferred glycol, with polypropyleneglycols having a weight average molecular weight of from 750 Da to 1,400 Da being particularly preferred.

Adjunct Ingredients

The composition may optionally comprise a number of other adjunct ingredients such as builders (preferably citrate), chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, viscosity adjusters (e.g., salt such as NaCl, and other mono-, di- and trivalent salts) and pH adjusters and buffering means (e.g. carboxylic acids such as citric acid, HCl, NaOH, KOH, alkanolamines, carbonates such as sodium carbonates, bicarbonates, sesquicarbonates, and alike).

Packaged product

The hand dishwashing detergent composition can be packaged in a container, typically plastic containers. Suitable containers comprise an orifice. Suitable containers include traditional upright dosing containers, where the orifice is at the top of the container, and inverted/bottom dosing containers, where the orifice is at the bottom of the container. For inverted/bottom dosing containers, the orifice may be capped and/or the orifice may comprise a slit valve, such as described in US Patent No. 10,611,531. Typically, the container comprises a cap, with the orifice typically comprised on the cap. The cap can comprise a spout, with the orifice at the exit of the spout. The spout can have a length of from 0.5 mm to 10 mm.

The orifice can have an open cross-sectional surface area at the exit of from 3 mm2 to 20 mm2, preferably from 3.8 mm2 to 12 mm2, more preferably from 5 mm2 to 10 mm2, wherein the container further comprises the composition according to the invention. The cross-sectional surface area is measured perpendicular to the liquid exit from the container (that is, perpendicular to the liquid flow during dispensing).

The container can typically comprise from 200 ml to 5,000 ml, preferably from 350 ml to 2000 ml, more preferably from 400 ml to 1,000 ml of the liquid hand dishwashing detergent composition.

Method of Washing

The invention is further directed to a method of manually washing dishware with the composition of the present invention. The method comprises the step of contacting the dishware with a composition according to the present invention.

Suitable methods can include the step of delivering a composition of the present invention to a volume of water to form a wash solution and immersing the dishware in the solution, in order to contact the dishware with the composition of the present invention. The dishware is then cleaned with the composition in the presence of water.

The dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware is contacted with an effective amount, typically from about 0.5 mL to about 20 mL (per about 25 dishes being treated), preferably from about 3 mL to about 10 mL, of the composition, preferably in liquid form, of the present invention diluted in water. The actual amount of composition used will be based on the judgment of the user and will typically depend upon factors such as the particular product formulation of the composition, including the concentration of active ingredients in the composition, the number of soiled dishes to be cleaned, the degree of soiling on the dishes, and the like. Generally, from about 0.01 mL to about 150 mL, preferably from about 3 mL to about 40 mL of a composition of the invention is combined with from about 2,000 mL to about 20,000 mL, more typically from about 5,000 mL to about 15,000 mL of water in a sink. The soiled dishware is immersed in the sink containing the diluted compositions then obtained, before contacting the soiled surface of the dishware with a cloth, sponge, or similar cleaning implement. The cloth, sponge, or similar cleaning implement may be immersed in the composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Alternatively, the composition herein can be applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user (immediately) prior to application. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90: 10 to 100:0 depending on the user habits and the cleaning task.

TEST METHODS

Polymer biodegradation

Biodegradation in wastewater was tested in triplicate using the OECD 301F manometric respirometry method. OECD 301F is an aerobic test that measures biodegradation of a sample by measuring the consumption of oxygen. To a measured volume of medium, 100 mg/L test substance, which is the nominal sole source of carbon is added along with the inoculum (30 mg/L, aerated sludge taken from Mannheim wastewater treatment plant). This is stirred in a closed flask at a constant temperature (20°C or 25°C) for 28 or 56 days, respectively. The consumption of oxygen is determined by measuring the change in pressure in the apparatus using an OxiTop® C (Xylem 35 Analytics Germany Sales GmbH & Co KG). Evolved carbon dioxide is absorbed in a solution of sodium hydroxide. Nitrification inhibitors are added to the flask to prevent usage of oxygen due to nitrification. The amount of oxygen taken up by the microbial population during biodegradation of the test substance (corrected for uptake by blank inoculum, run in parallel) is expressed as a percentage of ThOD (Theoretical oxygen demand, which is measured by the elemental analysis of the compound). A positive control Glucose/Glucosamine is run along with the test samples for each cabinet.

Alkoxylated polyol calculated molecular weight % PO, % EO, % polyol core

The calculated molecular weight (MW) can be derived from the molecular weights and amounts of the starting materials added during the reaction. Hence, the molecular weight (MW) of the alkoxylated polyol can be calculated using the equation below:

$$\text{Molecular weight (MW) of the alkoxylated polyol} =$$

$$[\text{Mass of polyol} + \text{mass of propylene oxide added} + \text{mass of ethylene oxide added} + \text{total}$$

$$\text{mass of other monomers (if used)}] / [\text{mass of polyol} / \text{molecular weight of the polyol}].$$

In the above, the mass of the polyol added and the molecular weight of the polyol refer to the mass of the monomer, oligomer or polymer, whichever is used.

For example, if during the reaction, 1,000 g of glycerine (92.09 g/mol) was added as the polyol core, in addition to 22,705 g of propylene oxide (58.08 g/mol) and 7,175g of ethylene oxide (44.05 g/mol), the molecular weight of the resultant alkoxylated polyol would be:

$$[1000 + 22705 + 7175] / [1000 / 92.09] = 2843.7 \text{ g/mol}.$$

From the masses of the added polyol, propylene oxide, ethylene oxide, and their molecular weights, the formula of the alkoxylated polyol can then be calculated as: $(\text{Glycerol})_1/[(\text{PO/OH})_{12}/(\text{EO/OH})_5]_3$, where in $(\text{polyol core})_1[(\text{PO/OH})_x(\text{EO/OH})_y]_m$, x refers to the average number of PO groups on each hydroxyl position of the polyol core, and y refers to the average number of EO groups on each hydroxyl position of the polyol core; m is the number of OH groups on the polyol used for the polyol core.

The % by weight of PO is then [58.08 x 12 x 3 x 100] / [2843.7] = 73.52%
The % by weight of EO is then [44.05 x 5 x 3 x 100] / [2843.7] = 23.23%
The % by weight of polyol core is then [92.09 x 100] / [2843.7] = 3.24%

Suds mileage index in the presence of greasy soil

Suds mileage performance in the presence of greasy soil was evaluated using the following method:
The objective of the Suds Mileage Index test is to compare the evolution over time of suds volume generated for different test formulations at specified water hardness, solution temperatures and formulation concentrations, while under the influence of periodic soil injections. Data are compared and expressed versus a reference composition as a suds mileage index (reference composition has suds mileage index of 100). The steps of the method are as follows:

1) A defined amount of a test composition, depending on the targeted composition concentration (0.12 wt%), is dispensed through a plastic pipette at a flow rate of 0.67 mL/ sec at a height of 37 cm above the bottom surface of a sink (dimension: 300 mm diameter and 288 mm height) into a water stream (using water at 1.25 mmol/l Ca equivalence (7dH) water hardness at 42°C) that is filling up the sink to 4 L with a constant pressure of 4 bar.

2) An initial suds volume generated (measured as average foam height X sink surface area and expressed in $cm^3$) is recorded immediately after end of filling.

3) A fixed amount (6 mL) of soil is immediately injected into the middle of the sink.

4) The resultant solution is mixed with a metal blade (10 cm x 5 cm) positioned in the middle of the sink at the air liquid interface under an angle of 45 degrees rotating at 85 RPM for 20 revolutions.

5) Another measurement of the total suds volume is recorded immediately after end of blade rotation.

6) Steps 3-5 are repeated until the measured total suds volume reaches a minimum level of 400 $cm^3$. The amount of added soil that is needed to get to the 400 $cm^3$ level is considered as the suds mileage for the test composition.

7) Each test composition is tested 4 times per testing condition (i.e., water temperature, composition concentration, water hardness, soil type).

8) The average suds mileage is calculated as the average of the 4 replicates for each sample.

9) Calculate a Suds Mileage Index by comparing the average mileage of a test composition sample versus a reference composition sample. The calculation is as follows:

$$\text{Suds Mileage Index} = \frac{\text{Average number of soil addition of test composition}}{\text{Average number of soil addition of reference composition}} \times 100$$

The soil composition is produced through standard mixing of the components described in 1able 1.

Table 1: Greasy soil used for the suds mileage test.

|  | wt % |
| --- | --- |
| Crisco® oil | 12.730 |
| Crisco® shortening | 27.752 |
| Lard | 7.638 |
| Refined rendered edible beef tallow | 51.684 |
| Oleic acid, 90% (Techn) | 0.139 |
| Palmitic acid, 99+% | 0.036 |
| Stearic acid, 99+% | 0.021 |

Viscosity measurement

The viscosity is measured using a controlled stress rheometer (such as an HAAKE MARS from Thermo Scientific, or equivalent), using a 60 mm 1° cone and a gap size of 52 microns at 20°C. After temperature equilibration for 2 minutes, the sample is sheared at a shear rate of 10 $s^{-1}$ for 30 seconds. The reported viscosity of the liquid hand dishwashing detergent compositions is defined as the average shear stress between 15 seconds and 30 seconds shearing divided by the applied shear rate of 10 $s^{-1}$ at 20°C.

pH:

The pH is measured as a 10 wt% solution in demineralized water at 20 °C, unless specified otherwise.

EXAMPLES

The following alkoxylated polyols were prepared as follows:

$(Glycerol)_1/[(PO/OH)_{12}/(EO/OH)_5]_3$ [IE 2, glycerol, alkoxylated with 12 moles propylene oxide per hydroxy group and 5 moles ethylene oxide per hydroxy group]:

Firstly, glycerol propoxylated with 4 moles propylene oxide per hydroxy group was prepared as follows: 100.0 g glycerol and 1.7 g potassium tert-butoxide were placed in a 2L autoclave and the mixture heated to 80°C. The autoclave vessel was purged three times with nitrogen and the mixture was heated to 140°C. 756.8 g propylene oxide was added in a continuous stream over 10 hours. The mixture was allowed to post-react for an additional 6 hours at 140°C in order to complete the reaction. The reaction mixture was stripped with nitrogen and volatile compounds were removed in vacuo at 80°C. After filtration 901.0 g of a light brown oil was obtained. The hydroxyl number was measured to be 208.1 mg KOH/g.

159.7 g of the resultant glycerol, propoxylated with 4 moles propylene oxide per hydroxy group and 0.8 g potassium tert-butoxide were placed in the 2L autoclave and the mixture was heated to 80°C. The vessel was purged three times with nitrogen and the mixture was heated to 140°C. 278.8 g propylene oxide was added continuously over 4 hours. The mixture was allowed to post-react for additional 4 hours at 140°C to complete the reaction. 132.1 g ethylene oxide was added over 2 hours, and the mixture allowed to post-react for an additional 10 hours. The reaction mixture was stripped with nitrogen and volatile compounds were removed in vacuo at 80°C. After filtration 565.0 g of a light brown oil was obtained. Elemental composition was 59.5 % carbon, 29.3 % oxygen, 10.5 % hydrogen, determined by pyrolysis / IR detection (oxygen) and combustion analysis / GC detection (carbon / hydrogen).

Other inventive and comparative examples were prepared in a similar manner. The structures are summarized in Table 1.

Table 1: Alkoxylated polyols of use in inventive (IE) and comparative (CE) liquid hand dishwashing detergent compositions.

| | Structure | wt% PO[a] | MW[b] | Biodegradability (28 days) |
|---|---|---|---|---|
| IE 1 | (Trimethylolpropane)$_1$/[(PO/OH)$_{12}$/(EO/OH)$_5$]$_3$ | 72.5 | 2886 | 86 |
| IE 2 | (Glycerol)$_1$/[(PO/OH)$_{12}$/(EO/OH)$_5$]$_3$ | 73.5 | 2844 | 88 |
| IE 3 | (Glycerol)$_1$/[(PO/OH)$_{15}$/(EO/OH)$_5$]$_3$ | 77.6 | 3366 | 90 |
| IE 4 | (Glycerol)$_1$/[(PO/OH)$_{20}$/(EO/OH)$_5$]$_3$ | 82.2 | 4238 | 86 |
| IE 5 | (1,2,4-Butantriol)$_1$/[(PO/OH)$_{12}$/(EO/OH)$_5$]$_3$ | 73.2 | 2858 | 93 |
| IE 6 | (1,2,4-Butantriol)$_1$/[(PO/OH)$_{15}$/(EO/OH)$_5$]$_3$ | 77.3 | 3380 | 90 |
| IE 7 | (1,2,6-Butantriol)$_1$/[(PO/OH)$_{12}$/(EO/OH)$_5$]$_3$ | 72.5 | 2886 | 86 |
| IE 8 | (Meso-erythritol)$_1$/[(PO/OH)$_{12}$/(EO/OH)$_5$]$_4$ | 73.5 | 3791 | 77 |
| IE 9 | (Meso-erythritol)$_1$/[(PO/OH)$_{15}$/(EO/OH)$_5$]$_4$ | 77.6 | 4488 | 59 |
| IE 10 | (Meso-erythritol)$_1$/[(PO/OH)$_{15}$/(EO/OH)$_7$]$_4$ | 57.8 | 3214 | 50 |
| IE 11 | (Meso-erythritol)$_1$/[(PO/OH)$_{15}$/(EO/OH)$_3$]$_4$ | 84.3 | 4136 | 66 |
| IE 11 | (1,2,5,6-Hexantetrol)$_1$/[(PO/OH)$_8$/(EO/OH)$_7$]$_4$ | 57.3 | 3243 | 57 |
| IE 12 | (Meso-erythritol)$_1$/[(EO/OH)$_5$/(PO/OH)$_{12}$]$_4$ | 73.5 | 3791 | 63 |
| IE 13 | (Pentaerythritol)$_1$/[(PO/OH)$_{12}$/(EO/OH)$_5$]$_4$ | 73.5 | 3805 | 71 |
| IE 14 | (Trimethylolpropane)$_1$/[(PO/OH)20/(EO/OH)$_5$]$_3$ | 81.4 | 4280 | 80 |
| CE A* | (Glycerol)$_1$/[(PO/OH)$_{12}$]$_3$ | 95.8 | 2183 | 90 |
| CE B* | (Meso-erythritol)$_1$/[(PO/OH)$_{15}$]$_4$ | 96.6 | 3607 | 80 |
| CE C* | (Meso-erythritol)$_1$/[(PO/OH)$_{17.5}$]$_4$ | 97.1 | 4188 | 81 |
| CE D* | (Meso-erythntol)$_1$/[(PO/OH)$_7$/(EO/OH)$_{10}$]$_4$ | 46.3 | 3510 | 10 |
| CE E* | (Meso-erythritol)$_1$/[(PO/OH)$_{24}$/(EO/OH)$_{16}$]$_4$ | 46.1 | 8068 | 7 |
| CE F* | (Meso-erythritol)$_1$/[(EO/OH)$_{10}$/(PO/OH)$_7$]$_4$ | 46.3 | 3510 | 7 |
| CE G* | (Trispentaerythritol)$_1$/(PO/OH)$_{12}$/(EO/OH)$_5$]$_8$ | 72.3 | 7710 | 15 |
| CE H* | (Pentaerythritol)$_1$/[(PO/OH)$_{12}$/(EO/OH)$_5$]$_4$ | 82.0 | 5664 | 8 |
| [a] propylene oxide content by weight% in relation to the total weight of the alkoxylated polyol [b] calculated molecular weight, based on the molar ratios of the starting materials | | | | |

The following liquid hand dishwashing compositions were prepared by simple mixing. All the examples comprised the same level of surfactant and the same ratio of anionic surfactant to co-surfactant.

Inventive examples 1 to 4 comprised 2% by weight of an alkoxylated polyol derived from either trimethylpropane or glycerol polyol in which the hydroxyl groups have been substituted with alkylene oxide branches, each alkylene comprises ethylene oxide and propylene oxide, wherein the polyol was first propoxylated and then ethoxylated.

In contrast, comparative example A comprised an alkoxylated polyol derived from a glycerol polyol in which the hydroxyl groups have been substituted with alkylene oxide branches, each alkylene comprises propylene oxide units only. Comparative example B did not comprise an alkoxylated polyol.

Table 2: Comparative and inventive liquid hand dishwashing detergent compositions.

| Wt% (100% active basis) | Ex 1 wt% | Ex 2 wt% | Ex 3 wt% | Ex 4 wt% | Ex A* wt% | Ex B* wt% |
|---|---|---|---|---|---|---|
| C12-13 AE0.6S[1] | 15.36 | 15.36 | 15.36 | 15.36 | 15.36 | 15.36 |
| C12-14 dimethyl amine oxide | 5.91 | 5.91 | 5.91 | 5.91 | 5.91 | 5.91 |
| Alkoxylated polyol IE 1[2] | 2.0 | - | - | - | - | - |
| Alkoxylated polyol IE 2[3] | - | 2.0 | - | - | - | - |
| Alkoxylated polyol IE 3[4] | - | - | 2.0 | - | - | - |
| Alkoxylated polyol IE 4[5] | - | - | - | 2.0 | - | - |
| Alkoxylated polyol CE A[6] | - | - | - | - | 2.0 | - |
| NaCl | 0.5 | 0.5 | 0.5 | 0.5 | 0.5 | 0.5 |
| Na Cumene Sulphonate (NaCS) | 0.5 | 0.5 | 0.5 | 0.5 | 0.5 | 0.5 |
| Polypropylene glycol (MW 1000) | 0.4 | 0.4 | 0.4 | 0.4 | 0.4 | 0.4 |
| Ethanol | 0.5 | 0.5 | 0.5 | 0.5 | 0.5 | 0.5 |
| Proxel® BIT preservative | 0.005 | 0.005 | 0.005 | 0.005 | 0.005 | 0.005 |
| Phenoxyethanol | 0.08 | 0.08 | 0.08 | 0.08 | 0.08 | 0.08 |
| Water and minors (dye, perfume) | bal. | bal. | bal. | bal. | bal. | bal. |
| | | | | | | |
| pH | 9 | 9 | 9 | 9 | 9 | 9 |
| Polymer biodegradibility | 84 | 89 | 89 | 85 | 90 | n.a. |
| Suds mileage index under greasy conditions 42°C, 1.25 mmol/l Ca equivalence (7dH)[+] | 110 | 112 | 111 | 104 | 98 | ref |
| * Comparative<br>[+] Index vs. comparative example B<br>[1] 42.06% branching<br>[2] $(Trimethylolpropane)_1/[(PO/OH)_{12}/(EO/OH)_5]_3$<br>[3] $(Glycerol)_1/[(PO/OH)_{12}/(EO/OH)_5]_3$<br>[4] $(Glycerol)_1/[(PO/OH)_{15}/(EO/OH)_5]_3$<br>[5] $(Glycerol)_1/[(PO/OH)_{20}/(EO/OH)_5]_3$<br>[6] $(Glycerol)_1/[(PO/OH)_{12}]_3$ | | | | | | |

From comparing the biodegradability of the polymers of the alkoxylated polyols used in inventive examples 1 to 4, and the suds mileage under greasy conditions form the compositions comprising them, with that of comparative example A, it can be seen that the propoxylated ethoxylated polyols of use in the present invention provide good biodegradability, while providing improved suds mileage. Comparative A, while also providing good biodegradability, demonstrates inferior suds mileage performance.

Inventive examples 5 to 7 comprised 2% by weight of an alkoxylated polyol derived from a meso-erythritol polyol in which the hydroxyl groups have been substituted with alkylene oxide branches, each alkylene comprises ethylene oxide and propylene oxide, wherein the polyol was first propoxylated and then ethoxylated. The inventive compositions all comprise an alkoxylated polyol in which the propylene oxide content was at least 50 % by weight in relation to the total weight of the alkoxylated polyol.

In contrast, comparative example C comprised an alkoxylated polyol in which the propylene oxide content was less than 50 % by weight in relation to the total weight of the alkoxylated polyol. Comparative examples D and E comprised alkoxylated polyols derived from a meso-erythritol polyol in which the hydroxyl groups have been substituted with alkylene oxide branches, each alkylene comprises propylene oxide units only. Comparative example F did not comprise an alkoxylated polyol.

Table 3: Comparative and inventive liquid hand dishwashing detergent compositions.

| Wt% (100% active basis) | Ex 5 wt% | Ex 6 wt% | Ex 7 wt% | Ex C* wt% | Ex D* wt% | Ex E* wt% | Ex F* wt% |
|---|---|---|---|---|---|---|---|
| C12-13 AE0.6S anionic[1] | 17.33 | 17.33 | 17.33 | 17.33 | 17.33 | 17.33 | 17.33 |
| C12-14 dimethyl amine oxide | 3.93 | 3.93 | 3.93 | 3.93 | 3.93 | 3.93 | 3.94 |
| Alkoxylated polyol IE 8[7] | 2 | - | - | - | - | - | - |
| Alkoxylated polyol IE 9[8] | - | 2 | - | - | - | - | - |
| Alkoxylated polyol IE 10[9] | - | - | 2 | - | - | - | - |
| Alkoxylated polyol CE B[10] | - | - | - | 2 | - | - | - |
| Alkoxylated polyol CE C[11] | - | - | - | - | 2 | - | - |
| Alkoxylated polyol CE D[12] | - | - | - | - | - | 2 | - |
| NaCl | 0.5 | 0.5 | 0.5 | 0.5 | 0.5 | 0.5 | 0.5 |
| NaCS | 0.5 | 0.5 | 0.5 | 0.5 | 0.5 | 0.5 | 0.5 |
| Polypropylene glycol (MW 1000) | 0.4 | 0.4 | 0.4 | 0.4 | 0.4 | 0.4 | 0.4 |
| Proxel® BIT preservative | 0.005 | 0.005 | 0.005 | 0.005 | 0.005 | 0.005 | 0.005 |
| Phenoxyethanol | 0.08 | 0.08 | 0.08 | 0.08 | 0.08 | 0.08 | 0.08 |
| Water and minors (dye, perfume) | Bal | Bal | Bal | Bal | Bal | Bal | Bal |
| | | | | | | | |
| pH (10% solution in demi water) | 9 | 9 | 9 | 9 | 9 | 9 | 9 |
| EO | 5 | 5 | 7 | 0 | 0 | 10 | - |
| PO | 12 | 15 | 8 | 15 | 17.5 | 7 | - |
| Biodegradibility | 77 | 59 | 50 | 80 | 81 | 10 | n.a. |
| Suds mileage index under greasy conditions 42°C, 1.25 mmol/l Ca equivalence (7dH)[++] | 113 | 113 | 111 | 103 | 101 | 120 | ref |

[++] Index vs. comparative example F
[7] $(Meso\text{-}erythritol)_1/[(PO/OH)_{12}/(EO/OH)_5]_4$
[8] $(Meso\text{-}erythritol)_1/[(PO/OH)_{15}/(EO/OH)_5]_4$
[9] $(Meso\text{-}erythntol)_1/[(PO/OH)_8/(EO/OH)_7]_4$
[10] $(Meso\text{-}erythritol)_1/[(PO/OH)_{15}]_4$
[11] $(Meso\text{-}erythritol)_1/[(PO/OH)_{17.5}]_4$
[12] $(Meso\text{-}erythritol)_1/[(PO/OH)_7/(EO/OH)_{10}]_4$

From Table 3, it can be seen that the compositions of inventive examples 5 to 7 and comparative example E, comprising an alkoxylated polyol derived from a polyol in which the hydroxyl groups have been substituted with alkylene oxide branches, each alkylene comprises ethylene oxide and propylene oxide, provide improved suds mileage under greasy conditions, over the compositions of comparative compositions C and D, in which the polyol has been propoxylated alone. From comparing the biodegradability of the alkoxylated polyols used in inventive examples 5 to 7 with the biodegradability of the alkoxylated polyol used in comparative example E, it can be seen that alkoxylated polyols in which the propylene oxide content was at least 50 % by weight in relation to the total weight of the alkoxylated polyol, provide improved biodegradability to alkoxylated polyols having a lower weight fraction of propoxylation.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A liquid hand dishwashing detergent composition comprising:

   a. from 5% to 50% by weight of the composition of a surfactant system, wherein the surfactant system comprises anionic surfactant; and
   b. an alkoxylated polyol derived from a polyol core having essentially 3 to 5 -OH groups, wherein:

   i. at least one of the -OH groups is modified to form an alkylene oxide branch;
   ii. each of the at least one alkylene oxide branches comprises ethylene oxide and propylene oxide;
   iii. the weight average molecular weight (Mw) of the alkoxylated polyol is in the range of from 1.500 to 4.500 g/mol; and
   iv. the propylene oxide content is at least 50% by weight in relation to the total weight of the alkoxylated polyol.

2. The composition according to claim 1, wherein the polyol core is a monomer, oligomer or polymer, wherein each of the oligomer and the polymer comprise a plurality of subunits, preferably the oligomer is a homooligomer or the polymer is a heteropolymer.

3. The composition according to any preceding claim, wherein each of the alkylene oxide branches comprise, preferably consist of, on average:

   a. at least 2 ethylene oxide units (EOs), preferably at least 3 EOs and more preferably at least 4 EOs; and
   b. at least 6 polypropylene oxide units (POs), preferably at least 7 POs and more preferably at least 8 POs.

4. The composition according to any preceding claim, wherein each of the alkylene oxide branches comprise, preferably consist of, on average:

   a. not more than 12 EOs, preferably not more than 10 EOs and more preferably not more than 8 EOs; and
   b. not more than 25 POs, preferably not more than 17 POs and more preferably not more than 14 POs.

5. The composition according to any preceding claim, wherein the polyol core has a molecular weight ranging from 80 to 500 g/mol, preferably from 100 to 300 g/mol and more preferably from 120 to 250 g/mol.

6. The composition according to any preceding claim, wherein the weight average molecular weight (Mw) of the alkoxylated polyol is in the range of from 2.000 to 4.300 g/mol, preferably in the range of from 2.500 to 4.000 g/mol, more preferably in the range of from 3.000 to 3500 g/mol.

7. The composition according to any preceding claim, wherein the polyol core is selected from the group consisting of: trimethylolpropane, glycerol, meso-Erythritol, D-threitol, L-threitol, 1,2,5,6-hexanetetrol, pentaerythritol, xylitol, ribitol, arabitol, pentitol, diglycerol, triglycerol, and mixtures thereof.

8. The composition according to any preceding claim, wherein each of the alkylene oxide branches comprise a block or random structure of ethylene oxide and propylene oxide, preferably a block structure, more preferably wherein each of the alkylene oxide branches are first propoxylated and then ethoxylated such that the propoxylation is closer to the polyol core.

9. The composition according to any preceding claim, wherein the polyol core has essentially 4 to 5 -OH groups, preferably 4 -OH groups.

10. The composition according to any preceding claim, wherein the alkoxylated polyol is present at a level of from 0.05% to 5.0%, preferably from 0.1% to 3.5%, more preferably from 0.3% to 2.5% by weight of the composition.

11. The liquid hand dishwashing detergent composition according to any one of the preceding claims, wherein the composition comprises from 6.0% to 40%, preferably from 15% to 35%, by weight of the detergent composition of the surfactant system.

12. The liquid hand dishwashing detergent composition according to any one of the preceding claims, wherein the surfactant system comprises at least 40%, preferably from 50% to 90%, more preferably from 65% to 85% by weight of the surfactant system of the anionic surfactant,
preferably wherein the anionic surfactant comprises at least 70%, preferably at least 85%, more preferably 100% by weight of the anionic surfactant of alkyl sulfated anionic surfactant, preferably the alkyl sulfated anionic surfactant has a number average alkyl chain length of from 8 to 18 carbon atoms, preferably from 10 to 14 carbon atoms, more preferably from 12 to 14 carbon atoms, even more preferably from 12 to 13 carbon atoms.

13. The liquid hand dishwashing detergent composition according to claim 12, wherein the alkyl sulfated anionic surfactant has an average degree of alkoxylation of less than 3.5, preferably less than 2.0, more preferably 1.0 or less.

14. The liquid hand dishwashing detergent composition according to any of claims 12 or 13, wherein the anionic surfactant comprises branched anionic surfactant, more preferably wherein the anionic surfactant has a weight average degree of branching of at least 10%, preferably from 20% to 60%, more preferably from 25% to 45%.

15. The liquid hand dishwashing detergent composition according to preceding claim, wherein the composition further comprises a co-surfactant selected from amphoteric surfactant, zwitterionic surfactant, and mixtures thereof, preferably wherein the anionic surfactant and the amphoteric co-surfactant are present in a weight ratio of from 1:1 to 8:1, more preferably from 2:1 to 5:1, most preferably from 2.5:1 to 4:1, preferably wherein the co-surfactant comprises amphoteric surfactant, wherein the amphoteric surfactant comprises amine oxide amphoteric co-surfactant, more preferably wherein the amine oxide amphoteric co-surfactant is selected from the group consisting of: alkyl dimethyl amine oxide, alkylamidopropyl dimethyl amine oxide, and mixtures thereof.

Europäisches
Patentamt

European
Patent Office

Office européen
des brevets

## EUROPEAN SEARCH REPORT

**Application Number**

EP 24 16 6335

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP S62 106999 A (KAO CORP) 18 May 1987 (1987-05-18) * compositions 22, 23, 31-34; claims 1-2; example 3; tables 4, 5 * | 1-15 | INV. C11D3/37 |
| X | US 4 492 646 A (WELCH JOHN B [US]) 8 January 1985 (1985-01-08) * example V * | 1-8, 10-15 | |
| X | EP 0 222 557 A2 (PROCTER & GAMBLE [US]) 20 May 1987 (1987-05-20) * column 11, line 37 - line 44; claims 1-3, 6, 7, 15, 20, 21, 22; example XIV * * page 5, line 41 - line 42 * | 1-8, 10-15 | |
| X | JP 4 810844 B2 (NOF CORP) 9 November 2011 (2011-11-09) * paragraph [0020] - paragraph [0023]; examples a3, 3; table 2 * * paragraph [0025] - paragraph [0028] * | 1-5,7,8, 10-13,15 | |
| A | EP 3 456 805 A1 (PROCTER & GAMBLE [US]) 20 March 2019 (2019-03-20) * claims 1-7, 15 * | 11-15 | TECHNICAL FIELDS SEARCHED (IPC) C11D |

The present search report has been drawn up for all claims

| Place of search | Date of completion of the search | Examiner |
|---|---|---|
| The Hague | 4 November 2024 | Loiselet-Taisne, S |

EPO FORM 1503 03.82 (P04C01)

**ANNEX TO THE EUROPEAN SEARCH REPORT
ON EUROPEAN PATENT APPLICATION NO.**

EP 24 16 6335

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2024

| Patent document cited in search report | | Publication date | Patent family member(s) | | Publication date |
|---|---|---|---|---|---|
| JP S62106999 | A | 18-05-1987 | JP | H0232318 B2 | 19-07-1990 |
| | | | JP | S62106999 A | 18-05-1987 |
| US 4492646 | A | 08-01-1985 | NONE | | |
| EP 0222557 | A2 | 20-05-1987 | AU | 589225 B2 | 05-10-1989 |
| | | | CA | 1299962 C | 05-05-1992 |
| | | | DE | 3689165 T2 | 05-05-1994 |
| | | | DK | 522786 A | 01-05-1987 |
| | | | EP | 0222557 A2 | 20-05-1987 |
| | | | FI | 864424 A | 01-05-1987 |
| | | | MX | 165143 B | 29-10-1992 |
| JP 4810844 | B2 | 09-11-2011 | JP | 4810844 B2 | 09-11-2011 |
| | | | JP | 2006249146 A | 21-09-2006 |
| EP 3456805 | A1 | 20-03-2019 | EP | 3456805 A1 | 20-03-2019 |
| | | | WO | 2019055255 A1 | 21-03-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7938900 B2
- CN 111518631 A
- JP 7189610 A
- WO 2019173688 A
- EP 3505609 A
- JP 6955746 B
- CN 108690742 A
- CN 106833953 A
- WO 2016191238 A
- ES 2464872 A
- JP 5394133 A
- JP 5324207 A

- JP 2008156250 A
- JP 5213092 A
- WO 2008021971 A
- JP 4554498 A
- JP 4810844 A
- JP 4810843 A
- WO 2021165468 A
- EP 3298120 A
- JP 2022056680 A
- US 7468348 B
- US 10611531 B

**Non-patent literature cited in the description**

- **ABRAHAM, D. S.** Production of propylene oxide from propylene glycol. *Master's Thesis University of Missouri-Columbia*, 2007, 75

- Determination of the Number-Average Molecular Weight and the Molecular Weight Distribution of Polymers using Gel Permeation Chromatography. OECD Guidelines for the Testing of Chemicals. OECD Publishing, 1996, 2074-5753